(19) Europäisches Patentamt
European Patent Office
Office européen des brevets

(11) Publication number: **0 405 602 A1**

# (12) EUROPEAN PATENT APPLICATION

(21) Application number: **90112500.5**

(22) Date of filing: **29.06.90**

(51) Int. Cl.[5]: **C07D 209/86**, C07D 209/88, C07C 317/44, C07C 59/64, C07C 229/42, A61K 31/00

(30) Priority: **30.06.89 ES 8902318**
**18.09.89 ES 8903152**
**08.06.90 ES 9001590**

(43) Date of publication of application:
**02.01.91 Bulletin 91/01**

(84) Designated Contracting States:
**AT BE CH DE DK FR GB IT LI NL SE**

(71) Applicant: **LABORATORIOS VINAS S.A.**
**386, Calle Provenza**
**E-08025 Barcelona(ES)**

(72) Inventor: **Buxadé, Antonio**
**230, Calle Compte d'Urgell**
**E-08036 Barcelona(ES)**

(74) Representative: **Dipl.-Ing. Schwabe, Dr. Dr. Sandmair, Dr. Marx**
**Stuntzstrasse 16**
**D-8000 München 80(DE)**

## (54) New Zinc derivatives of anti-inflammatory drugs having improved therapeutic activity.

(57) New zinc derivatives of anti-inflammatory drugs having an improved therapeutical activity, particularly new Formula (I) zinc derivatives, a process for the preparation thereof, a pharmaceutical composition

$$(W\text{-}(CR^1R^2)_q\text{-}COO^{(-)})_mZn^{(+2)} \quad (I)$$

containing them and a method for using these zinc derivatives in anti-inflammatory therapy.

EP 0 405 602 A1

# NEW ZINC DERIVATIVES OF ANTI-INFLAMMATORY DRUGS HAVING IMPROVED THERAPEUTIC ACTIVITY

## Technical field of the invention

This invention relates to new zinc derivatives of non-steroidal anti-inflammatory drugs. More particularly it relates to new zinc derivatives of Formula (I) having anti-inflammatory and mucosa protecting activity.

$(W\text{-}CR^1R^2)_q\text{-}COO^{(-)})_mZn^{(+2)}$ (I)

This invention also relates to processes for preparing the compounds of Formula (I) as well as to a pharmaceutical composition containing anyone of the compounds of Formula (I) as active ingredient.

## Background of the invention

Zinc is an essential trace element for living beings and is highly important in physiological processes and therefore in human pathology.

The non-steroidal anti-inflammatory drugs form a very wide range of compounds having a great variety of chemical groups and as such, or in salt, fundamentally sodium salt form, are known for their anti-inflammatory and antirheumatic properties. In spite of this important therapeutical action, nevertheless, gastric toxicity of varying degree depending on the drug is one of the most frequent side effects (British Medical Journal, 292, 1190, 1986) and, consequently, this toxicity is a limitation of said therapeutical agents. Therefore, research directed to removing or reducing the side effects of these therapeutical agents, particularly the toxic action on the gastric mucosa, without their anti-inflammatory therapeutical action being diminished, is of interest. Even more guaranteed is the interest of such research if it is considered that treatment with antiacids or antisecretory agents has not been able to prevent such effects, and particularly to treat cases in which these effects occur in patients suffering acute anti-inflammatory or rheumatic processes in which suppression of the therapy is not advisable.

## Description of the invention

An object of this invention is new zinc derivatives of Formula (I) as new compounds *per se* .

A further object of the patent is non-steroidal anti-inflammatory zinc derivatives having anti-inflammatory and antirheumatic activity and mucosa protecting activity.

A further object of the invention is a process for the preparation of said Formula (I) derivatives.

Yet another object of this invention is a pharmaceutical composition containing any of the zinc derivatives of Formula (I) as active ingredient.

Yet a further object of the invention is a method, foreseeably useful, for treating patients needing anti-inflammatory therapy, comprising administering to such patients, by way of an adequate composition, an effective amount of any of the zinc derivatives of Formula (I) containing a sufficient amount of zinc for the mucosa protective action to be manifested with a view to reducing or preventing the undesired effects of the anionic moiety.

This invention relates to new zinc derivatives of anti-immflamatory drugs of Formula (I)

$(W\text{-}CR^1R^2)_q\text{-}COO^{(-)})_mZn^{(+2)}$ (I)

where:

*m* may be 2 or 1, depending on whether the total number or carboxyl groups in the organic fraction is one or two respectively;

*q* may be 0 (formic derivatives are thus formed), 1 (acetic derivatives), 2 (propionic derivatives) or 3 (butyric derivatives). Also when *q* is 2 or 3, the carbons may be bonded together by a double link to form vinyl or acrylic derivatives;

*W* may be a cyclic or heterocyclic fragment, and may be represented by any of the formulas A, B, C, D and E

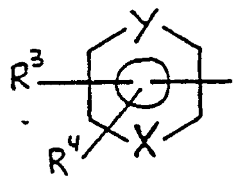

A

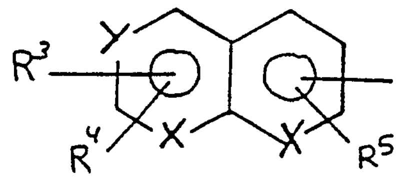

B

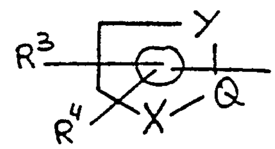

C

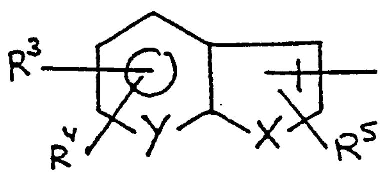

D

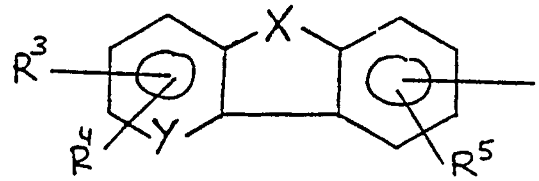

E

where:

X , Y and Q may be the same or different simultaneously;

X is: =CH-, $=CR^3$, $=CR^4$, $=CR^5$, > C =CH-$R^6$ ( cis or trans ), -NH- (for example forming indoles in D or carbozoles in E ), =N-;

Y and Q are: =CH-, $=CR^3$, $=CR^4$, $=CR^5$, =N-, -NH-, >N-$R^7$, -O-, -S-.

For the purposes of making formulas A, B, C, D and E more intelligible, X and Y have been shown in particular positions; nevertheless, the relative positions between X and Y may be 1,2; 1,3; 1,4 and 1,5.

When X and Y are both =CH-, A and B will be benzene and naphthalene, respectively.

$R^1$, $R^2$, $R^3$, $R^4$, $R^5$, $R^6$ and $R^7$ may be the same or different and indifferently may be: hydrogen, halogen (fluor, chlorine, bromine or iodine), carboxyl groups, straight or branched chain lower ($C_1$-$C_5$) alkyl groups (isopropyl, 2-methylpropyl, etc.), straight or branched chain lower ($C_1$-$C_5$) alkoxy groups (isopropyl, 2-methylpropionyl, etc.) aryl, aryloxy, alkoyl, aroyl or heterocyclic ring. The alkyl or alkoxy groups may be totally or partially replaced with halogens (example, trifluoromethyl, 2,2,2-trifluoroethyl, etc.).

$R^3$, $R^4$ and $R^5$ may be attached to the ring or heterocycle in different positions relative to the $((CR^1R^2)_q$-$COO^{-})$ group of Formula (I),

$R^3$, $R^4$ and $R^5$ may also be attached directly to the A, B, C, D and E systems, or through a Z group, $R^3$-then being $R_1{}^3Z$-, $R^4$- being $R_1{}^4Z$- and $R^5$- being $R_1{}^5Z$- and in these cases $R_1{}^3$, $R_1{}^4$ and $R_1{}^5$ are the same as $R^3$-, $R^4$ and $R^5$ respectively. Z may be: -CH-, $-(CH_2)_p-$ (where p may be 0, 1, 2), -NH, -N-, -CO-, -O-, -S-.

In turn the alkyl, aryl, alkoxy, aryloxy, alkoyl and aroyl groups and the heterocyclic compounds may have one or two halogen groups, straight or branched chain lower alkyl ($C_1$-$C_5$) groups (isopropyl, 2-methylpropionyl, etc.), straight chain lower ($C_1$-$C_4$) or branched chain ($C_1$-$C_4$) alkyl sulphinyl groups, straight or branched chain lower alkoxy ($C_1$-$C_5$) groups (isopropyl, 2-methylpropionyl, etc.) or primary or secondary amino groups. The alkyl or alkoxy groups may be totally or partially replaced with by halogens (trifluoromethyl, 2,2,2-trifluorethyl, etc.).

According to the invention, the new Formula (I) compounds, unlike other salts of the corresponding anti-inflammatory drugs, or of the free acids, have improved therapeutical properties, that is, an anti-inflammatory-antirheumatic activity and a gastric mucosa protective activity. This additional protective activity is particularly important since in extended treatments with patients suffering from chronic arthritic processes, a compulsory interruption of the treatment is frequent as result of the toxic effects on the gastric mucosa.

Another foreseeable advantage of the Formula (I) compounds is the possibility of using them for the prevention of said side effects, without having to wait for these to appear.

Furthermore, it is also important to highlight that the dose of any of the Formula (I) compounds should be substantially considered in respect with the amount of zinc to be administered. Thus, it will be based on a daily zinc dose of above 7 mg and less than 150 mg, with the purpose of manifesting the protective effect. It should also be considered that within the said zinc range, the corresponding anion must be present in a sufficient amount to make its anti-inflammatory action effective.

Finally, a further advantage of these new Formula (I) compounds is that they allow the two molecular

fractions providing the therapeutic activity to be conjugated in a single active ingredient, thereby avoiding the administration of other ions, physiological or otherwise, but which need for that reason be administered, such us sodium, chlorides, sulphates, carboxylic acids, etc.

The process for preparing the new Formula (I) compounds of the present invention is characterized by reacting the corresponding acids of Formula (II):

**$(W\text{-}CR^1R^2)_q\text{-}COOH$**

(where q , W , $R^1$ and $R^2$ have the same meaning given hereinbefore) with zinc oxide, hydroxide, carbonates or salts, or reacting a salt of the corresponding acids, which may be ammonium, alkali or alkaline-earth salts, with a zinc salt such as the chloride, nitrate, sulphate, phosphate, acetate, etc.

The reactant may be present in equimolar amounts and also in excess or in deficiency relative to substrate.

Preferably there is used an alkali or ammonium salt (as such or formed in situ ) and equimolar amounts of zinc chloride or nitrate.

The reaction may be conducted in polar solvents or a mixture of polar solvents, preferably in water or in a mixture of water and low molecular weight alcohols such as methanol, ethanol, isopropanol, etc.

The reaction temperature may range from 0 °C up to the reflux temperature of the solvent or of the mixture of solvents, preferably between room temperature and the reflux temperature of the solvent or mixture of solvents.

The zinc reactant may be added as a solid, in portions; dissolved in the same solvent when said reactant is soluble; and in paste form or in suspension in the same solvent.

The compound may be isolated by crystallisation or by precipitation. It will subsequently be filtered and dried.

Examples of preparation

Hereinafter there are described several examples of the process for preparing the compounds of Formula (I) of the invention:

**Example 1: Preparation of zinc (2-(6-chlorocarbazol-2-yl)propionate) of Formula (III), abbreviated to LV-236**

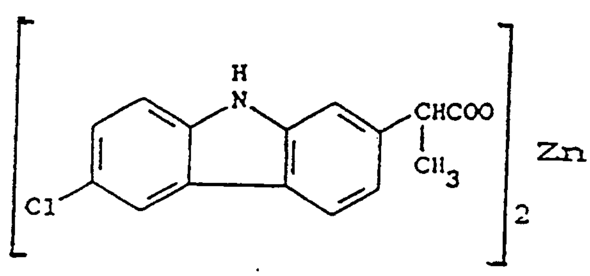

(III)

108 ml of 0.1 N sodium hydroxide and 580 ml of water were added to 3.0 g (11,0 mmoles) of 2-(6-chlorocarbazol-2-yl) propionic acid. The thus obtained suspension was heated to approximately 65 °C to achieve dissolution of the acid.

In parallel, there was prepared a further solution by dissolving 1.50 g (11.0 mmoles) of anhydrous zinc chloride in 150 ml water and this solution was slowly added over the previous one. At the end of the addition the reaction is allowed to stand with stirring for an hour at 65 °C, after which the solid was filtered, washed and allowed to dry at room temperature for 24 hours (or down to constant weight).

The product obtained by this process, a white or ivory white solid, was in sufficiently pure crystalline form so as not to require subsequent purification.

By drying at room temperature it is obtained as a dihydrate (KF or loss on dessication) which may be dehydrated under vacuum and/or high temperature, nevertheless, this hydrates very rapidly whereby it is preferable to obtain it and identify as dihydrate.

Melting point: > 310 °C

IR (KBr): 3413, 2987, 1549, 1462, 1420, 1336, 1275, 1245, 1062, 872 and 582 $cm^{-1}$.

| Elementary analysis for $C_{30}H_{26}CL_2N_2O_6Zn$: | | | | |
|---|---|---|---|---|
| | C | H | N | Zn |
| Calculated (%) | 55,71 | 4,05 | 4,33 | 10,11 |
| Found (%) | 55,34 | 4,24 | 4,38 | 10,15 |

**Example 2: Preparation of zinc (2-(6-chlorocarbazol-2-yl)propionate) of Formula (III), abbreviated to LV-236**

50 ml of ethanol, 50 ml of water and 133 mg (0.60 mmoles) of zinc acetate dihydrate are added over 500 mg (1.8 mmoles) of 2-(6-chlorocarbazol-2-yl )propionic acid. The reaction mixture was stirred at 80 °C for several hours (4-10 hours), after which it was filtered and the solid was washed with an ethanol/water mixture and allowed to dry at room temperature.

There was thus obtained a white solid having the same characteristics as that of the previous example.

**Example 3: Preparation of zinc (2-(6-chlorocarbazol-2-yl)propionate) of Formula (III), abbreviated to LV-236**

100 ml of water and 45 mg (0.55 mmoles) of zinc oxide were added over 400 mg (1.4 mmoles) of 2-(6-chlorocarbazol-2-yl)propionic acid. The reaction mixture was stirred under reflux for several hours (4-10 hours), after which it was allowed to cool to 60 °C and 100 ml of ethanol were added while maintaining the stirring and the temperature to between 40-60 °C for a further 30 minutes. The mixture is filtered and the solid was washed with an ethanol/water mixture and allowed to dry at room temperature.

Thus a white solid was obtained having the same characteristics as that of the previous example.

**Example 4: Preparation of zinc (Z)-(5-fluoro-2-methyl-1- (4-methylsulphinylbenzylidene)inden-3-yl)-acetate of Formula (IV), abbreviated to LV-237**

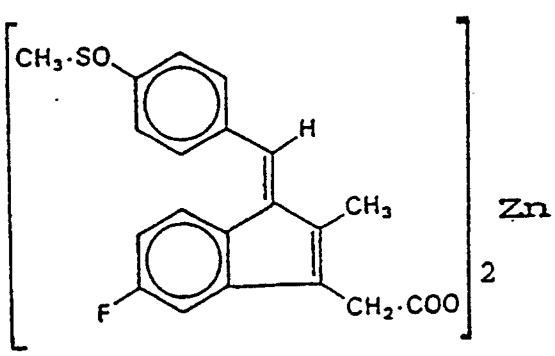

(IV)

10 ml (1.0 mmol) of 0.1 N sodium hydroxide and 50 ml water were added over 357 mg (1.0 mmol) of (Z)-(5-fluoro-2-methyl-1-(4-methylsulphinylbenzylidene) inden-3-yl) acetic acid. The mixture was heated to 60 °C and there was added thereto a solution of 70 mg (0.5 mmoles) of anhydrous zinc chloride in 25 ml of water. At the end of the addition, the reaction was held under stirring for one hour at the same temperature, after which it was filtered, the solid was washed and allowed to dry at room temperature for 24 hours (or down to constant weight).

A hydrated yellow solid was thus obtained.

Melting point: 181-185 °C

IR (KBr): 3424, 1602, 1466, 1400, 1272, 1170, 1028, 1010 and 859 $cm^{-1}$.

Water content 6.5-7.5 %. Zinc content 7.9-8.1 %. Zinc determination in anhydrous product: 8.3-8.5 %

(theoretical value 8.42 %).

**Example 5: Preparation of zinc (1-(4-chlorobenzoyl-5-methoxy-2-methylindol-3-yl)) acetate of Formula (V), abbreviated to LV-238**

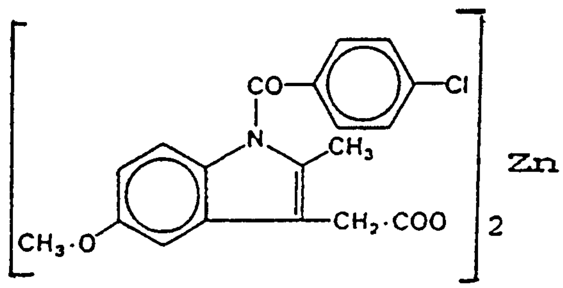

(V)

140 ml (14.0 mmoles) of 0.1 N sodium hydroxide and 400 ml of water were added over 5.00 g (14.0 mmoles) of (1-(4-chlorobenzoyl)-5-methoxy-2-methylindol-3-yl)) acetic acid. The mixture was heated to 60 °C to dissolve the acid and a solution of 0.76 g (5.6 mmoles) of anhydrous zinc chloride in 150 ml of water was added. When the addition was over, the reaction mixture was stirred for one hour at same temperature, after which it was filtered, the solid was washed and allowed to dry at room temperature for 24 hours (or down to constant weight).

In this way a white hydrated solid having the following characteristics was obtained:
Melting point: 227-233 °C
IR (KBr): 3384, 2935, 1677, 1588, 1476, 1401, 1354, 1329, 1233, 1150, 916 and 756 $cm^{-1}$.

| Elementary analysis for $C_{38}H_{34}Cl_2N_2O_{10}Zn$: | | | |
|---|---|---|---|
| | C | H | Zn |
| Calculated (%) | 56,00 | 4,21 | 8,02 |
| Found (%) | 55,74 | 4,54 | 7,98 |

**Example 6: Preparation of zinc 2-((3-(trifluoromethyl)phenyl)-amino)benzoate of Formula (VI) abbreviated to LV-239**

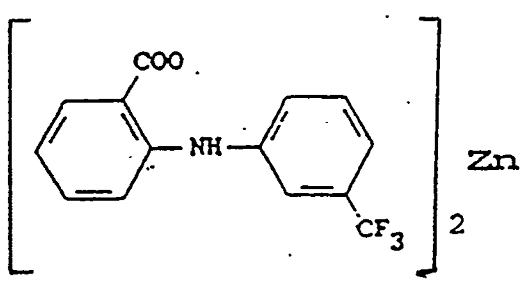

(VI)

219 ml (21.9 mmoles) of 0.1 N sodium hydroxide and 400 ml of water were added over 6.16 g (21.9 mmoles) of 2-((3-(trifluoromethyl)-phenyl)-amino)benzoic acid. The mixture was heated to 60 °C and a solution of 1.50 g (11.0 mmoles) of anhydrous zinc chloride in 150 ml of water was added. When the addition was over, the reaction mixture was stirred for one hour at the same temperature, after which it was filtered, the solid was washed and allowed to dry at room temperature for 24 hours (or down to constant

weight).

In this way a white or slightly yellowish solid was obtained. When dried over phosphorous pentoxide at reduced pressure and at 80 °C, it shows the following characteristics:

Melting point: 162-166 °C

IR (KBr): 3315, 1609, 1585, 1508, 1400, 1332, 1278, 1167, 1121, 927 and 790 $cm^{-1}$.

| Elementary analysis for $C_{28}H_{18}F_6N_2O_4Zn$: | | | |
|---|---|---|---|
| | C | H | Zn |
| Calculated (%) | 53,73 | 2,90 | 11,45 |
| Found (%) | 54,08 | 3,22 | 10,57 |

<u>Example 7: Preparation of zinc (+)-2-(6-(methoxy-2-naphthyl)propionate of Formula (VII) abbreviated to LV-218</u>

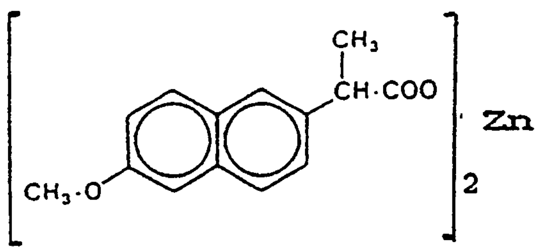

(VII)

17.5 ml of 1 N sodium hydroxide were added over a suspension of 4.0 g, (17.4 mmoles) of (+)-2-(6-(methoxy-2-naphthyl)propionic acid in 300 ml of water. The solution was heated to 60 °C and when all acid was dissolved, a solution of 1.18 g (8.7 mmoles) of zinc chloride in 100 ml of water was added. When the addition was over, the stirring and temperature were maintained for one hour, after which the mixture was cooled, filtered and the solid was washed and dried at 40 °C.

In this way a white or ivory coloured crystalline solid was obtained. The zinc analysis (11.75%, complexometry), water determination (6.5%, K.F. or dessication at 110 °C) and comparison with the analytical results of an anhydrous sample, show that it is a dihydrate.

The compound, after dehydration at 80 °C at reduced pressure in the presence of phosphorous pentoxide showed the following analytical characteristics:

Melting point: 234-236 °C

IR (KBr): 2936, 1634, 1606, 1550, 1460, 1423, 1392, 1268, 1032 and 853 $cm^{-1}$

U.V. max. (0. 1N NaOH): 262±2; 272±2 nm $(\alpha)_D^{22}$ = +55±3o (c=2, 4% ethanol in phosphoric acid)

| Elementary analysis for $C_{28}H_{26}O_6Zn$: | | | |
|---|---|---|---|
| | C | H | Zn |
| Calculated (%) | 64,19 | 5,00 | 12,48 |
| Found (%) | 63,96 | 4,98 | 12,55 |

<u>Example 8: Preparation of zinc (+)-2-(6-(methoxy-2-naphthyl)propionate of Formula (VII), abbreviated to LV-218</u>

1.5 g (15.0 mmoles) of zinc hydroxide were added over a solution of 2.3 g (10.0 mmoles) of (+)-2-(6-

methoxy-2-naphthyl)propionic acid in 300 ml of 96.5° ethanol and the mixture was held under reflux for an hour. It was filtered and the filtrate was allowed to cool and crystallize. The solid was filtered, washed and allowed to dry at 40° C.

In this way a white crytalline solid was obtained and after drying at 80° C, at reduced pressure in the presence of phosphorous pentoxide, it showed the same analytical characteristics as those of the compound obtained in Example 7.

**Example 9: Preparation of zinc 2-(3-benzoylphenyl)propionate of Formula (VIII), abbreviated to LV-219**

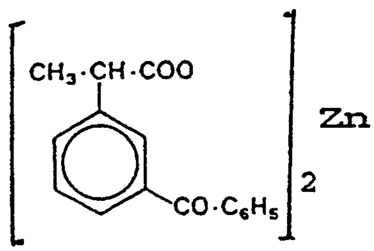

(VIII)

20 ml of 1N sodium hidroxide were added over a suspension of 5.1 g (20.0 mmoles) of 2-(3-benzoylphenyl)propionic acid in 250 ml of distilled water. Once the acid was completely dissolved, a further solution of 1.37 g (10.0 mmoles) of zinc chloride in 150 ml of water was added over the former solution slowly. Once the addition was over, the reaction mixture was stirred for an hour and filtered, the solid was washed with water and allowed to dry at reduced pressure in the presence of phosphorous pentoxide. Once dry, after grinding, an ivory white coloured solid having the following analytical characteristics was obtained:

IR (KBr): 3061, 1657, 1596, 1415, 1318, 1283, 718, and 644 $cm^{-1}$

U.V. max. (0.1N NaOH): 263±2; 220±2 nm

| Elementary analysis for $C_{32}H_{26}O_6Zn$: | | | |
|---|---|---|---|
| | C | H | Zn |
| Calculated (%) | 64,19 | 5,00 | 12,48 |
| Found (%) | 63,96 | 4,98 | 12,55 |

**Pharmacological activity**

As described, an anti-inflammatory activity and protective activity are conjugated in the Formula (I) compounds prepared according to the examples described herein: LV-218, LV-219, LV-236, LV-237, LV-238, LV-239.

It is highlighted that the protective activity in rats against ethanol induced ulcer at doses between 5 and 250 mg/kg of these new compounds reached inhibition levels of up to 85%:

| | dose (mg/kg) | max inhibition (%) |
|---|---|---|
| LV-218 | 100-250 | 85 |
| LV-219 | 10-30 | 50 |
| LV-236 | 5-15 | 85 |
| LV-237 | 25-50 | 70 |
| LV-238 | 5-15 | 41 |
| LV-239 | 25-50 | 54 |

The following tests more explicitly show the test results showing the pharmacological activity of these compounds:

**A) Anti-inflammatory effect**

The anti-inflammatory activity of different Formula (I) compounds was studied in the carrageenin edema described by Winter et al. (Proc. Soc. Exp. Biol. Med. 111:544, 1962), using Wistar rats having a body weight between 150 and 200 g. The inflammation was induced by intradermal injection of a carrageenin suspension in the paw pad and the volume of the paw was measured by plethysmography. The anti-inflammatory effect was evaluated calculating the percentage inhibition of inflammation in the treated groups versus the control.

The results obtained with different Formula (I) compounds show high inflammation inhibition levels as per the following table at the doses given.

| | dose (mg/kg) | inhibición (%) |
|---|---|---|
| (+)-2-(6-(methoxi-2-naphtyl)-propionic acid<br>LV-218 | 150<br>177 | 73<br>78 |
| 2-(3-benzoylfenyl)-propiónic acid<br>LV-219 | 20<br>22 | 55<br>61 |
| 2-(6-chlorocarbazol-2-yl)-propionic acid<br>LV-236 | 10<br>12 | 44<br>45 |
| (Z)-(5-fluoro-2-methyl-1-(4-methylsulfinylbencilidene)-inden-3-yl) acetic acid<br>LV-237 | 30<br>34 | 50<br>48 |
| (1-(4-chlorobenzoyl)-5-methoxy-2-methylindol-3-yl)) acetic<br>LV-238 | 10<br>12 | 47<br>44 |
| 2-((3-(trifluoromethyl)-phenyl)-amino)benzoic acid<br>LV-239 | 30<br>33 | 56<br>55 |

**B) Cytoprotective effect**

Using the experimental model of absolute ethanol induced ulcers described by Robert et al. (Gastroenterology 77: 433, 1979), the cytoprotective activity of the known zinc derivatives was compared also with the corresponding acids at equimolar doses and a control group which received the vehicle.

The following Table gives the lesion indexes (en ulcerated mm) of the groups studied, as well as the inhibition percentages of the ulcers of the treated group versus the control (mean values ± the error of the mean).

| | dose (mg/kg) | lesión (mm) | inhibición (%) |
|---|---|---|---|
| Control | | 61,5±9,6 | --- |
| (+)-2-(6-(methoxi-2-naphthyl)propiónic acid | 150 | 73,5±4,4 | 0 |
| LV-218 | 177 | 28,8±7,5[a,b] | 53,2 |
| Control | | 118,1±5,9 | --- |
| 2-(3-benzoylphenyl)-propiónic acid | 20 | 104,0±4,5 | 11,9 |
| LV-219 | 22 | 83,4±9,3[a,b] | 29,4 |
| Control | | 112,3±8,5 | --- |
| 2-(6-chlorocarbazol-2-yl)propiónic acid | 10 | 85,0±96 | 24,3 |
| LV-236 | 12 | 46,1±11,8[a,b] | 58,9 |
| Control | | 95,2±7,6 | --- |
| (Z)-(5-fluoro-2-methyl-1-(4-metilsulphinylbenzylidene)-inden-3-yl)-acétic acid | 30 | 89,3 = 3±6,5 | 6,1 |
| LV-237 | 34 | 58,9±9,7[a,b] | 38,2 |
| Control | | 102,0±8,7 | --- |
| (1-(4-chlorobenzoyl)-5-methoxy-2-methylindol-3-yl))-acétic acid | 10 | 104,7±5,4 | 0 |
| LV-238 | 12 | 83,6±7,3[a,b] | 17,9 |
| Control | | 102,0±10,2 | --- |
| 2-((3-(trifluoromethyl)-phenil)-amino)-benzoic acid | 30 | 98,4±6,5 | 8,3 |
| LV-239 | 33 | 74,9±8,2[a,b] | 30,1 |
| t test: | | | |

[a] p<0.001 vs. control;
[b] p<0.5 vs. acid.

As may be seen, some of the corresponding acids inhibit, to a certain extent, the absolute ethanol induced ulcers, while with the zinc derivatives the inhibition is superior and statistically significant relative to the control and the group treated with the corresponding acids.

## Pharmaceutical forms

For therapeutical use, the new Formula (I) zinc compounds of the present invention will normally be administered in pharmaceutical forms comprising any one of said compounds alone or in association with a pharmaceutical vehicle as essential active ingredient.

The pharmaceutical vehicle used may be, for example, a solid, a liquid or a mixed vehicle. Examples of solid vehicles are: lactose, terra alba, saccharose, talc, gelatine, agar, pectin, acacia, magnesium stearate, stearic acid and the like. Examples of liquid vehicles are: syrup, groundnut oil, olive oil, water and the like.

Consequently, it will be understood that a wide range of pharmaceutical forms may be used. Thus, if a solid vehicle is used, any of the Formula (I) compounds may be prepared as tablets, placed in hard gelatine capsules, powders, granules, tablets, etc.

If a liquid vehicle is used, the preparations with any of the active compounds of Formula (I) may take on the form of syrup, emulsion, soft gelatine capsules, sterile injectable liquid solution, as vial or in aqueous or non-aquous liquid suspension, etc.

When mixed vehicles are used, any of the Formula (I) compounds may be offered in form of creams, ointments, suppositories, etc.

By any of the types of solid, liquid or mixed pharmaceutical vehicles mentioned, the active compounds of the invention may be also be formulated in retarded oral or topical forms.

The pharmaceutical forms are prepared by the conventional galenic techniques in which there are used processes such as mixing, granulating and pressing, dispersion or disolution of the ingredients as appropriate for the desired preparation.

The active compounds will be present in the pharmaceu tical form in an effective amount allowing from 7 to 150 mg of zinc per day to be supplied, depending on the particular zinc derivative selected, on the conditions of each patient and the physician's criteria. There is no limit to the administration route, which may be, among others, oral, parenteral, rectal, topical, etc.

Other pharmacologically active compounds may be included in one same pharmaceutical form.

## Claims

1.- Zinc derivatives of general Formula (I)

$$\mathbf{(W\text{-}CR^1R^2)_q\text{-}COO^{(-)})_mZn^{(+2)}} \quad (I)$$

where:

m , may be 2 or 1, depending on whether the total number or carboxyl groups in the organic fraction is one or two respectively;

q may be 0 (formic derivatives are thus formed), 1 (acetic derivatives), 2 (propionic derivatives) or 3 (butyric derivatives), and also when q is 2 or 3, the carbons may be bonded together by a double link to form vinyl or acrylic derivatives;

W may be a cyclic or heterocyclic fragment, and may be represented by any of the formulas A, B, C, D and E

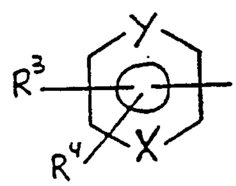

A

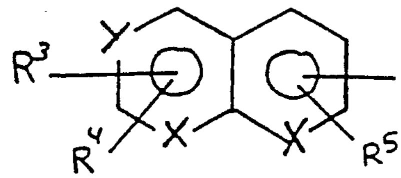

B

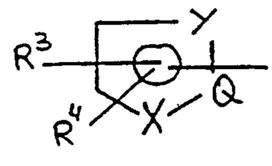

C

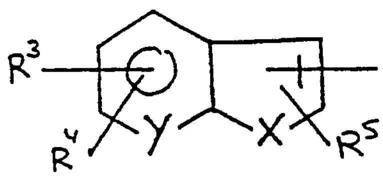

D

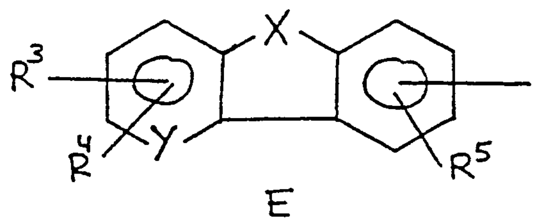

E

where:

- nevertheless, the relative positions between X and Y may be 1,2; 1,3; 1,4 and 1,5.
- X , Y and Q may be the same or different simultaneously;
- X is: $=CH-$, $=CR^3$, $=CR^4$, $=CR^5$, $>C$ $CH-R^6$ ( cis or trans ), -NH-, =N-;
- Y and Q are: $=CH-$, $=CR^3$, $=CR^4$, $=CR^5$, =N-, -NH-, $>N-R^7$, -O-, -S-;
- when X and Y are both =CH-, A and B are benzene and naphthalene, respectively;
- $R^1$, $R^2$, $R^3$, $R^4$, $R^5$, $R^6$ and $R^7$ may be the same or different and indifferently may be: hydrogen, halogen (fluor, chlorine, bromine or iodine), carboxyl groups, straight or branched chain lower ($C_1$-$C_5$) alkyl groups, straight or branched chain lower ($C_1$-$C_5$) alkoxy groups aryl, aryloxy, alkoyl, aroyl or a heterocyclic ring;
- the alkyl or alkoxy groups may be totally or partially replaced with halogens;
- $R^3$, $R^4$ and $R^5$ may be attached to the ring or heterocycle in different positions relative to the $((CR^1R^2)_q$-$COO^{-})$ group of Formula (I);
- $R^3$, $R^4$ and $R^5$ may also be attached to the A, B, C, D and E systems, either directly or through a Z group, $R^3$- then being $R_1{}^3Z$-, $R^4$- being $R_1{}^4Z$- and $R^5$- being $R_1{}^5Z$-and in these cases $R_1{}^3$, $R_1{}^4$ and $R_1{}^5$ are the same as $R^3$, $R^4$ and $R^5$ respectively. Z may be: -CH-, $-(CH_2)_p$-(where p may be 0, 1, 2), -NH, -N-, -CO-, -O-, -S-;
- the said alkyl, aryl, alkoxy, ariloxy, alkoyl and aroyl groups and the heterocyclic compounds mentioned above may have one or two halogen groups, straight or branched chain lower alkyl ($C_1$-$C_5$) groups, straight chain lower ($C_1$-$C_4$) or branched chain ($C_1$-$C_4$) alkyl sulphinyl groups, straight or branched chain lower alkoxy ($C_1$-$C_5$) groups ( or primary or secondary amino groups;
- the alkyl or alkoxy groups may be totally or partially replaced with by halogens.

2.- The zinc derivatives of claim 1), wherein m is 2.

3.- The zinc derivatives of claims 1) and 2) wherein $R^1$ is methyl, $R^2$ is hydrogen and q is 1.

4.- The zinc derivatives of claims 1) and 2), wherein $R^1$ and $R^2$ are both hydrogen and q is 1.

5.- The zinc derivatives of claims 1) and 2) wherein q is 0.

6.- The zinc derivatives of claims 1), 2) and 3), wherein one compound is zinc 2-(6-chlorocarbazol-2-yl)-propionate.

7.- The zinc derivatives of claims 1), 2) and 3) wherein one compound is zinc 2-(3-benzoyl-phenyl)-propionate.

8.- The zinc derivatives of claims 1), 2) and 3), wherein the compound is zinc (+)-2-(6-methoxy-2-naph thyl)propionate.

9.- The zinc derivatives of claims 1), 2) and 4) wherein the compound is zinc (Z)-(5-fluoro-2-methyl-1-(4-methylsulphinyl-benzylidene)-inden-3-yl)acetate.

10.- A process for the preparation of new zinc derivatives of anti-inflammatory agents of the general Formula (I)

**$(W$-$CR^1R^2)_q$-$COO^{(-)})_mZn^{(+2)}$** (I)

(in which m , q , W , $R^1$ and $R^2$ have the same meaning as given in claim 1) wherein, in accordance therewhith, the corresponding acids of Formula (II):

**$(W$-$CR^1R^2)_q$-$COOH$** (II)

(in which q , W , $R^1$ and $R^2$ have the same meaning as given in claim 1) are reacted with zinc oxide, hydroxide, carbonates or salts or the corresponding salt of the acid (II), which may be ammonia, alkali or alkali earth, are reacted with a zinc salt such as chloride, nitrate, sul phate, phosphate, acetate, etc., the reaction taking place in a polar solvent or a mixture of polar solvents at a temperature lying between 0 °C and the reflux temperature of the solvent or solvent mixture.

11.- The process of claim 10), wherein said polar solvent is water and the mixture of solvents is water and low molecular weight alcohols.

12.- The process of claims 10) and 11), wherein the reaction temperature lies between room temperature and the reflux temperature of the solvent or solvent mixture.

13.- The process of claims 10), 11) and 12) wherein the zinc reactant is zinc oxide or hydroxide.

14.- The process of claims 10), 11) and 12) wherein the zinc salt is chloride, nitrate or acetate.

15.- The process of claims 10), 11), 12), 13) and 14) wherein the starting acid is 2-(6-chlorocarbazol-2-yl)-propionic acid and the compound obtained is zinc 2-(6-chlorocarbazol-2-yl )propionate.

16.- The process of claims 10), 11), 12), 13) and 14) wherein the starting acid is 2-(3-benzoylphenyl)-propionic acid and the compound obtained is zinc 2-(3-benzoylphenyl )propionate.

17.- The process of claims 10). 11), 12), 13) and 14) wherein the starting acid is (+)-2-(6-methoxy-2-naphthyl) propionic acid and the compound obtained is zinc (+)-2-(6-metoxy-2-naphtyl)propionate.

18.- The process of claims 10), 11), 12), 13) and 14) wherein the starting acid is (Z)-(5-fluoro-2-methyl-1-(4-methylsulphinyl-benzylidene)-inden-3-yl)acetic acid and the compound obtained is zinc (Z)-(5-fluoro-2-methyl-1-(4-methylsulphinyl-benzylidene)-inden-3-yl)acetate.

19.- A pharmaceutical compound having anti-inflammatory and gastric mucosa protective properties comprising an effective amount of any of the zinc compounds of claim 1) as active ingredient and a pharmaceutically acceptable vehicle.

20.- The pharmaceutical composition of claim 19, wherein the zinc compound is zinc 2-(6-chlorocarbazol-2-yl) propionate.

21.- The pharmaceutical composition of claim 19, wherein the zinc compound is zinc 2-(3-benzoylphenyl)-propionate.

22.- The pharmaceutical composition of claim 19, wherein the zinc compound is zinc (+)-2-(6-methoxy-2-naphthyl) propionate.

23.- The pharmaceutical composition of claim 19, wherein the zinc compound is zinc (Z)-(5-fluoro-2-methyl-1-(4-methylsulphinyl-benzylidene)-inden-3-yl)acetate.

24.- A method of treating patients needing anti-inflammatory therapy consisting of administering to such patients a pharmaceutical composition containing anyone of the zinc derivatives defined in claim 1) and according to claims 19), 20), 21), 22) or 23) in sufficient amounts to provide between 7 and 150 mg zinc per day.

European Patent Office

**PARTIAL EUROPEAN SEARCH REPORT**
which under Rule 45 of the European Patent Convention shall be considered, for the purposes of subsequent proceedings, as the European search report

Application number
EP 90112500.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A2 - 0 200 391 (AMERICAN HOME PRODUCTS CORPORATION) * Columns 1,2 * | 1,19 | C 07 D 209/86<br>C 07 D 209/88<br>C 07 C 317/44<br>C 07 C 59/64<br>C 07 C 229/42<br>A 61 K 31/00 |
| X | AT - B - 313 263 (MERCK & CO., INC) * Claim 1; page 3, lines 13-16 * | 1,2,4, 9,10, 19,24 | |
| A | DE - A1 - 3 040 737 (SELVI & C.S.P.A.) * Claim 1 * | 1,9 | |
| X | US - A - 3 737 455 (TSUNG-YING SHEN et al.) * Claim 1 * | 1,9 | |
| X | US - A - 4 009 197 (JOHN H. FRIED et al.) * Column 2, lines 52-57; column 10, lines 43-51 * | 1,8,10 | TECHNICAL FIELDS SEARCHED (Int. Cl.5) |
| X | DE - A1 - 3 445 011 | 1,4,19 | C 07 D 209/00<br>C 07 C |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-23
Claims searched incompletely: -
Claims not searched: 24
Reason for the limitation of the search: *(method for treatment of the human or animal body by therapy, Art. 52(4)EPC)*

| Place of search | Date of completion of the search | Examiner |
|---|---|---|
| VIENNA | 03-10-1990 | HEIN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82

European Patent Office

**PARTIAL EUROPEAN SEARCH REPORT**

Application number

-2-

EP 90112500.5

## DOCUMENTS CONSIDERED TO BE RELEVANT

| Category | Citation of document with indication, where appropriate, of relevant passages | Relevant to claim | CLASSIFICATION OF THE APPLICATION (Int Cl.5) |
|---|---|---|---|
| | (CIBA-GEIGY AG)<br>* Page 4; abstract *<br>-- | | |
| A | AT - B - 266 096<br>(J.R. GEIGY A.-G)<br>* Example 1; page 2, lines 41-44; page 1, lines 15-18 *<br>-- | 1,4,19 | |
| D,A | BRITISH MEDICAL JOURNAL, vol. 292, May 3, 1986<br>CSM UPDATE "Non-steroidal anti-inflammatory drugs and serious gastrointestinal adverse reactions-2"<br>page 1190<br>* Page 1190 *<br>---- | 1,19 | |

TECHNICAL FIELDS SEARCHED (Int. Cl.5)

EPO Form 1505.3 08.78